# EUROPEAN PATENT SPECIFICATION

(11) **EP 2 881 431 B1**
(45) Date of publication and mention of the grant of the patent: **26.06.2019**
(21) Application number: 13825499.0
(22) Date of filing: 01.08.2013
(51) Int. Cl.: A61K 8/24, A61Q 19/02, A61Q 19/10, C09D 4/00, C08K 3/10, C08K 5/00, C08K 5/092, C08F 220/18, C08K 3/32, A61K 8/365, A61K 8/67, A61K 8/81, C08K 5/098, A61Q 19/00

(54) **VISCOUS COMPOSITION**
VISKOSE ZUSAMMENSETZUNG
COMPOSITION VISQUEUSE

(30) Priority: 03.08.2012 JP 2012173447
(43) Date of publication of application: 10.06.2015
(73) Proprietor: Sumitomo Seika Chemicals Co., Ltd., Kako-gun Hyogo 675-0145 (JP)
(72) Inventor: MURAKAMI, Ryosuke, Himeji-shi Hyogo 672-8076 (JP); ASHIDA, Taro, Himeji-shi Hyogo 672-8076 (JP); MORIMITSU, Yuichiro, Himeji-shi Hyogo 672-8076 (JP)
(74) Representative: Mewburn Ellis LLP
(86) International application number: PCT/JP2013/070920
(87) International publication number: WO 2014/021434

(56) References cited:
- WO-A1-2007/055354
- WO-A1-2009/084469
- JP-A- H0 439 312
- JP-A- 2002 332 310
- JP-A- 2011 213 676

## Description

### TECHNICAL FIELD

The present invention relates to a viscous composition. More specifically, it relates to a viscous composition imparted with high viscosity over a pH range from acidic to weakly acidic conditions even while containing a carboxyl group-containing water-soluble copolymer in the presence of a polyvalent metal salt. Furthermore, the present invention relates to a method for producing the viscous composition.

### BACKGROUND ART

The carboxyl group-containing water-soluble copolymer has been used in various fields as a thickening agent for cosmetics or the like, a moisturizing agent including a cataplasm and the like, an emulsifying agent or a suspension stabilizer for a suspended substance or the like, a gelable base material for a battery or the like, and the like. In general, as a method for preparing a viscous composition increased in viscosity by means of a carboxyl group-containing water-soluble copolymer, a method of adding a carboxyl group-containing water-soluble copolymer to water and the like to prepare a homogeneous dispersion and then neutralizing the dispersion with an alkali to adjust the pH within a weakly acidic range has been known. By allowing various additives corresponding to respective applications to be blended into the water-containing viscous composition thus obtained, various products have been produced.

On the other hand, in various product categories, a polyvalent metal salt has been used as an additive. For example, magnesium ascorbyl phosphate, which is a polyvalent metal salt, has been used as a skin whitening agent, an anti-oxidizing agent or the like in the fields of cosmetic materials, medical and pharmaceutical products, and the like. However, in general, with regard to a carboxyl group-containing water-soluble copolymer, when the polyvalent metal salt exists, there is a problem that the viscosity is lowered because a part of the carboxyl group-containing water-soluble copolymer precipitates, and in the case of allowing the viscosity to be increased by means of a carboxyl group-containing water-soluble copolymer, the carboxyl group-containing water-soluble copolymer has a drawback that the kind of additives which can be blended therein is restricted.

On that account, as a thickening agent which enables a viscous composition with high viscosity to be formed even in the presence of the polyvalent metal salt, there has been reported an alkyl-modified carboxyl group-containing water-soluble copolymer with a specific structure (see Patent Literature 1). By means of the alkyl-modified carboxyl group-containing water-soluble copolymer disclosed in Patent Literature 1, a viscous composition allowing the viscosity to be increased to some extent in a pH range from acidic to weakly acidic conditions even in the presence of a polyvalent metal salt is obtained, but there has been an increase in demand for a product with further heightened viscosity and further improvement in the point of the enhancement in viscosity has been desired.

### PRIOR ART DOCUMENTS

### PATENT DOCUMENTS

Patent Document 1: WO 2007/055354 A

### SUMMARY OF THE INVENTION

### PROBLEM TO BE SOLVED BY THE INVENTION

An object of the present invention is to provide a viscous composition which is capable of achieving high viscosity over a pH range from acidic to weakly acidic conditions even in the presence of a polyvalent metal salt by means of an alkyl-modified carboxyl group-containing water-soluble copolymer.

### SOLUTIONS TO THE PROBLEM

The present inventors have conducted diligent studies in view of solving the above problems, whereupon they have found that a viscous composition obtained by adjusting the pH of a liquid composition containing a specific alkyl-modified carboxyl group-containing water-soluble copolymer, a polyvalent metal salt and water to 7.0 or greater with a base, and then, adjusting the pH to 2.5 to 6.5 with a carboxylic acid and/or a phosphorus oxo acid can be imparted with high viscosity. Furthermore, the present inventors have also found that, in the viscous composition, the stickiness which is a drawback peculiar to the alkyl-modified carboxyl group-containing water-soluble copolymer is suppressed, and the feeling of use at the time of being applied to a skin is remarkably enhanced. The present invention has been completed by further researches on the basis of such findings.

That is, the present invention provides a viscous composition of embodiments set forth below and a production method thereof.

Item 1. A viscous composition characterized as being obtained by adjusting the pH of a liquid composition containing an alkyl-modified carboxyl group-containing water-soluble copolymer obtained by allowing 100 parts by mass of a (meth)acrylic acid, 0.5 to 5 parts by mass of a (meth)acrylic acid alkyl ester having an alkyl group with 18 to 24 carbon atoms and 0 to 0.1 part by mass of a compound having two or more ethylenically unsaturated groups to undergo a polymerization, a polyvalent metal salt and water to 7.0 or greater with a base, and then, adjusting the pH to 2.5 to 6.5 with at least one kind of acid selected from the group consisting of a carboxylic acid and a phosphorus oxo acid.

The content of the alkyl-modified carboxyl group-containing water-soluble copolymer in the liquid composition is 0.1 to 4 parts by mass relative to 100 parts by mass of water.

Item 2. The viscous composition according to the item 1, wherein the polyvalent metal salt is a polyvalent metal salt of an organic acid.

Item 3. The viscous composition according to the item 1 or 2, wherein the polyvalent metal salt is a polyvalent metal salt of an ascorbic acid derivative.

Item 4. The viscous composition according to any one of the items 1 to 3, wherein the content of the alkyl-modified carboxyl group-containing water-soluble copolymer in the liquid composition is 0.1 to 3 parts by mass relative to 100 parts by mass of water. 3

Item 5. The viscous composition according to any one of the items 1 to 4, wherein the content of the polyvalent metal salt in the liquid composition is 0.1 to 4 parts by mass relative to 100 parts by mass of water.

Item 6. The viscous composition according to any one of the items 1 to 5, being a cosmetic material, an external preparation for skin, or a toiletry product.

Item 7. A viscous composition characterized as including (A) an alkyl-modified carboxyl group-containing water-soluble copolymer obtained by allowing 100 parts by mass of a (meth)acrylic acid, 0.5 to 5 parts by mass of a (meth)acrylic acid alkyl ester having an alkyl group with 18 to 24 carbon atoms and 0 to 0.1 part by mass of a compound having two or more ethylenically unsaturated groups to undergo a polymerization, (B) a polyvalent metal salt, (C) a base, (D) at least one kind of acid selected from the group consisting of a carboxylic acid and a phosphorus oxo acid, and (E) water, wherein the content of the alkyl-modified carboxyl group-containing water-soluble copolymer is 0.1 to 4 parts by mass relative to 100 parts by mass of water, the pH being 2.5 to 6.5, and
the degree of viscosity at 25°C being 1,000 to 100,000 mPa•s.

Item 8. The viscous composition according to the item 7, wherein the polyvalent metal salt is a polyvalent metal salt of an organic acid.

Item 9. The viscous composition according to the item 7 or 8, wherein the alkyl-modified carboxyl group-containing water-soluble copolymer, the polyvalent metal salt, the base, and the carboxylic acid and/or the phosphorus oxo acid are contained at respective contents of 0.1 to 4% by mass, 0.1 to 4% by mass, 0.01 to 6% by mass, and 0.1 to 4% by mass.

Item 10. The viscous composition according to any one of the items 7 to 9, being a cosmetic material, an external preparation for skin, or a toiletry product.

Item 11. A method for producing a viscous composition, including the following first to third steps of:
a first step for preparing a liquid composition containing an alkyl-modified carboxyl group-containing water-soluble copolymer obtained by allowing 100 parts by mass of a (meth)acrylic acid, 0.5 to 5 parts by mass of a (meth)acrylic acid alkyl ester having an alkyl group with 18 to 24 carbon atoms and 0 to 0.1 part by mass of a compound having two or more ethylenically unsaturated groups to undergo a polymerization, a polyvalent metal salt and water,
   wherein the content of the alkyl-modified carboxyl group-contaning water-soluble copolymer in the liquid composition is 0.1 to 4 parts by mass relative to 100 parts by mass of water,
a second step for preparing an alkaline viscous composition by adding a base to the liquid composition to adjust the pH to 7.0 or greater, and
a third step for preparing a viscous composition by adding at least one kind of acid selected from the group consisting of a carboxylic acid and a phosphorus oxo acid to the alkaline viscous composition to adjust the pH to 2.5 to 6.5.

Item 12. The method for producing a viscous composition according to the item 11, wherein the polyvalent metal salt is a polyvalent metal salt of an organic acid.

Item 13. The method for producing a viscous composition according to the item 11 or 12, wherein the polyvalent metal salt is a polyvalent metal salt of an ascorbic acid derivative.

Item 14. The method for producing a viscous composition according to any one of the items 11 to 13, wherein the content of the alkyl-modified carboxyl group-containing water-soluble copolymer in the liquid composition is 0.1 to 3 parts by mass relative to 100 parts by mass of water.

Item 15. The method for producing a viscous composition according to any one of the items 11 to 14, wherein the content of the polyvalent metal salt in the liquid composition is 0.1 to 4 parts by mass relative to 100 parts by mass of water.

### EFFECTS OF THE INVENTION

According to the present invention, since there can be provided a viscous composition with high viscosity by allowing the viscosity of an alkyl-modified carboxyl group-containing water-soluble copolymer to be highly increased over a pH range from acidic to weakly acidic conditions even in the presence of a polyvalent metal salt, it is possible to produce a formulation containing a polyvalent metal salt and having high viscosity in various product categories.

Moreover, in the case where the content of an alkyl-modified carboxyl group-containing water-soluble copolymer is large, although the conventional one has a drawback that a person has a feeling of stickiness and an unsatisfactory feeling of use when applied to a skin, according to the present invention, such a drawback has been eliminated, and it is possible to give no feeling of stickiness even when applied to a skin, and achieve a satisfactory feeling of use which gives a smooth dry feeling and a feeling of freshness.

### BRIEF DESCRIPTION OF THE DRAWINGS

Fig. 1 shows the results obtained by measuring the pH and the degree of viscosity during the period until a viscous composition (pH 5.0) is produced using a liquid composition (pH 4.5) in Example 14.
Fig. 2 shows the results obtained by measuring the pH and the degree of viscosity during the period until a viscous composition (pH 5.1) is produced using a liquid composition (pH 4.5) in Example 15.
Fig. 3 shows the results obtained by measuring the pH and the degree of viscosity during the period until a viscous composition (pH 5.3) is produced using a liquid composition (pH 2.5) in Example 16.

### DESCRIPTION OF EMBODIMENT

The viscous composition according to the present invention is characterized as being obtained by adjusting the pH of a liquid composition containing a specific alkyl-modified carboxyl group-containing water-soluble copolymer, a polyvalent metal salt and water to 7.0 or greater with a base, and then, adjusting the pH to 2.5 to 6.5 with a carboxylic acid and/or a phosphorus oxo acid. Hereinafter, the viscous composition according to the present invention will be described in detail.

### Alkyl-modified carboxyl group-containing water-soluble copolymer

The alkyl-modified carboxyl group-containing water-soluble copolymer used in the present invention is a polymer obtained by allowing 100 parts by mass of a (meth)acrylic acid, 0.5 to 5 parts by mass of a (meth)acrylic acid alkyl ester having an alkyl group with 18 to 24 carbon atoms and 0 to 0.1 part by mass of a compound having two or more ethylenically unsaturated groups to undergo a polymerization.

In this connection, in the present specification, "(meth)acrylic" means acrylic and/or methacrylic.

As the (meth)acrylic acid, any one of an acrylic acid and a methacrylic acid may be used alone or both of these may be used in combination.

The (meth)acrylic acid alkyl ester having an alkyl group with 18 to 24 carbon atoms refers to an ester of a (meth)acrylic acid and a higher alcohol having an alkyl group with 18 to 24 carbon atoms. Although such a (meth)acrylic acid alkyl ester is not particularly limited, examples thereof include an ester of a (meth)acrylic acid and stearyl alcohol, an ester of a (meth)acrylic acid and eicosanol, an ester of a (meth)acrylic acid and behenyl alcohol, an ester of a (meth)acrylic acid and tetracosanol, and the like. Of these (meth)acrylic acid alkyl esters, from the viewpoints of allowing the viscosity to be further heightened and allowing the feeling of use at the time of being applied to a skin to be enhanced in the viscous composition according to the present invention, examples thereof preferably include stearyl methacrylate, eicosanyl methacrylate, behenyl methacrylate, and tetracosanyl methacrylate. These (meth)acrylic acid alkyl esters each may be used alone or may be used in combination of two or more kinds thereof. Moreover, as such a (meth)acrylic acid alkyl ester, for example, a commercial product such as BLEMMER VMA70 as the trade name thereof available from NOF CORPORATION may be used.

The amount of the (meth)acrylic acid alkyl ester used is 0.5 to 5 parts by mass and preferably 1 to 3 parts by mass relative to 100 parts by mass of the (meth)acrylic acid. In the case where the amount of the (meth)acrylic acid alkyl ester used is less than 0.5 part by mass, at the time of allowing the resulting alkyl-modified carboxyl group-containing water-soluble copolymer to be dispersed in water, there is a tendency that an unmixed-in lump becomes easy to be generated. Moreover, in the case where the amount of the (meth)acrylic acid alkyl ester used is greater than 5 parts by mass, at the time of preparing a viscous composition with the resulting alkyl-modified carboxyl group-containing water-soluble copolymer, there is a tendency for the solubility of the alkyl-modified carboxyl group-containing water-soluble copolymer to become unsatisfactory.

Moreover, although the compound having two or more ethylenically unsaturated groups is not particularly limited, examples thereof include an acrylic acid ester prepared by allowing two or more hydroxyl groups in a polyol such as ethylene glycol, propylene glycol, polyoxyethylene glycol, polyoxypropylene glycol, glycerin, polyglycerin, trimethylolpropane, pentaerythritol, saccharose and sorbitol to be substituted; an allyl ether prepared by allowing two or more hydroxyl groups in the polyol to be substituted; diallyl phthalate, triallyl phosphate, allyl methacrylate, tetraallyloxyethane, triallyl cyanurate, divinyl adipate, vinyl crotonate, 1,5-hexadiene, divinylbenzene, polyallyl saccharose, and the like. Of these compounds having two or more ethylenically unsaturated groups, at the time of preparing a viscous composition with the resulting alkyl-modified carboxyl group-containing water-soluble copolymer, from the viewpoints of enabling the viscous composition to be imparted with high thickening properties with a smaller amount thereof to be used and enabling an emulsified substance, a suspended substance, or the like to be imparted with high suspension stability, examples thereof preferably include pentaerythritol allyl ether, tetraallyloxyethane, triallyl phosphate, and poly allyl saccharose. These compounds having two or more ethylenically unsaturated groups each may be used alone or may be used in combination of two or more kinds thereof.

The amount of the compound having two or more ethylenically unsaturated groups used is 0 to 0.1 part by mass and preferably 0.001 to 0.044 part by mass relative to 100 parts by mass of the (meth)acrylic acid. In the case where the amount of the compound having two or more ethylenically unsaturated groups used is greater than 0.1 part by mass, at the time of preparing a viscous composition with the resulting alkyl-modified carboxyl group-containing water-soluble copolymer, there is a tendency that an insoluble gel becomes easy to be produced.

In the production of the alkyl-modified carboxyl group-containing water-soluble copolymer, the method for polymerizing the (meth)acrylic acid, the (meth)acrylic acid alkyl ester and the optional compound having two or more ethylenically unsaturated groups is not particularly limited, and examples thereof include a method of allowing those to undergo a polymerization in a polymerization solvent in the presence of a radical polymerization initiator, and the like.

The radical polymerization initiator is not particularly limited, and examples thereof include α,α'-azobisisobutyronitrile, 2,2'-azobis-2,4-dimethylvaleronitrile, 2,2'-azobismethylisobutylate, benzoyl peroxide, lauroyl peroxide, cumene hydroperoxide, tertiary butyl hydroperoxide, and the like. These radical polymerization initiators each may be used alone or may be used in combination of two or more kinds thereof.

Although the amount of the radical polymerization initiator used is not particularly limited, for example, the amount is preferably 0.01 to 0.45 part by mass and more preferably 0.01 to 0.35 part by mass relative to 100 parts by mass of the (meth)acrylic acid. By using the radical polymerization initiator within the above-mentioned range, the polymerization reaction rate can be appropriately controlled and it is possible to economically produce the alkyl-modified carboxyl group-containing water-soluble copolymer.

Moreover, although the polymerization solvent is not particularly limited, it is preferred that the polymerization solvent is a solvent which dissolves the (meth)acrylic acid, the (meth)acrylic acid alkyl ester and the compound having two or more ethylenically unsaturated groups and does not dissolve the resulting alkyl-modified carboxyl group-containing water-soluble copolymer. Specific examples of such a polymerization solvent include normal pentane, normal hexane, normal heptane, normal octane, isooctane, cyclopentane, methylcyclopentane, cyclohexane, methylcyclohexane, benzene, toluene, xylene, chlorobenzene, ethylene dichloride, ethyl acetate, isopropyl acetate, ethyl methyl ketone, isobutyl methyl ketone, and the like. Of these polymerization solvents, from the viewpoints of being stable in quality and being easily available, examples thereof preferably include ethylene dichloride, normal hexane, normal heptane, and ethyl acetate. These polymerization solvents each may be used alone or may be used in combination of two or more kinds thereof.

Although the amount of the polymerization solvent used is not particularly limited, for example, the amount is preferably 200 to 10,000 parts by mass and more preferably 300 to 2,000 parts by mass relative to 100 parts by mass of the (meth)acrylic acid. By using the polymerization solvent within the above-mentioned range, even when the polymerization reaction proceeds, the alkyl-modified carboxyl group-containing water-soluble copolymer is suppressed from flocculating, the reaction liquid can be uniformly stirred, and the polymerization reaction can be allowed to efficiently proceed.

Although the atmosphere under which the above-mentioned polymerization reaction is performed is not particularly limited as long as the polymerization reaction is possible, examples thereof include an inert gas atmosphere such as nitrogen gas and argon gas.

Although the reaction temperature at which the above-mentioned polymerization reaction is performed is not particularly limited as long as the polymerization reaction is possible, for example, the reaction temperature is preferably 50 to 90°C and more preferably 55 to 75°C. By performing the polymerization reaction at such a reaction temperature, the viscosity increase of the reaction solution can be suppressed, the reaction control can be facilitated, and furthermore, the bulk density of the resulting alkyl-modified carboxyl group-containing water-soluble copolymer can be controlled.

Although the reaction time during which the above-mentioned polymerization reaction is performed cannot be decided sweepingly since the period of time varies depending on the reaction temperature, the reaction time is usually 2 to 10 hours.

After the completion of the reaction, for example, by heating the reaction solution to 80 to 130°C to remove the polymerization solvent, a finely powdered white alkyl-modified carboxyl group-containing water-soluble copolymer can be isolated.

In the viscous composition according to the present invention, the alkyl-modified carboxyl group-containing water-soluble copolymers each may be used alone or may be used in combination of two or more kinds thereof.

### Polyvalent metal salt

The polyvalent metal salt used in the present invention is not particularly limited as long as the polyvalent metal salt can liberate bi- or higher valent metal ions in an aqueous solution, and appropriately selected according to the application of the viscous composition according to the present invention.

Although the bi- or higher valent metal is not particularly limited, examples thereof include magnesium, calcium, strontium, barium, radium, zinc, aluminum and the like. Of these, magnesium and calcium can be suitably used in the present invention since magnesium and calcium are used as polyvalent metal salts in products belonging to various categories.

Specifically, examples of the polyvalent metal salt include a polyvalent metal salt of an inorganic acid and a polyvalent metal salt of an organic acid.

Specifically, examples of the polyvalent metal salt of an inorganic acid include magnesium chloride, magnesium bromide, magnesium iodide, magnesium sulfate, magnesium carbonate, calcium chloride, calcium bromide, calcium iodide, calcium sulfate, calcium carbonate, calcium phosphate, calcium pyrophosphate, and the like.

Specifically, examples of the polyvalent metal salt of an organic acid include magnesium lactate, magnesium acetate, magnesium citrate, magnesium pyrrolidone carboxylate, magnesium benzoate, monomagnesium di-L-glutamate, magnesium ascorbate, magnesium ascorbyl phosphate, magnesium sulfo-L-ascorbate, calcium lactate, calcium acetate, calcium citrate, calcium pyrrolidone carboxylate, calcium benzoate, calcium ascorbyl phosphate, calcium sulfo-L-ascorbate, and the like.

Of these polyvalent metal salts, examples thereof preferably include a polyvalent metal salt of an organic acid. Above all, a polyvalent metal salt of an ascorbic acid derivative such as magnesium ascorbyl phosphate, magnesium sulfo-L-ascorbate, calcium ascorbyl phosphate and calcium sulfo-L-ascorbate is suitably used in the present invention because the polyvalent metal salt of an ascorbic acid derivative is excellent in antioxidant action and has a high level of usefulness as an active component or an additive blended in a cosmetic material, medical and pharmaceutical products, or the like.

These polyvalent metal salts each may be used alone or may be used in combination of two or more kinds thereof.

### Liquid composition containing alkyl-modified carboxyl group-containing water-soluble copolymer, polyvalent metal salt and water

The preparation of the viscous composition according to the present invention is performed using a liquid composition containing an alkyl-modified carboxyl group-containing water-soluble copolymer, a polyvalent metal salt and water (sometimes written simply as "a liquid composition").

Although the content of the alkyl-modified carboxyl group-containing water-soluble copolymer in the liquid composition needs only to be appropriately set according to the viscosity to be imparted to the viscous composition according to the present invention, the content is 0.1 to 4 parts by mass, more preferably 0.1 to 3 parts by mass, and further preferably 0.2 to 2 parts by mass relative to 100 parts by mass of water. When the content lies within such a range, the feeling of use at the time when the viscous composition according to the present invention is applied to a skin can also be further improved.

Moreover, although the content of the polyvalent metal salt in the liquid composition also needs only to be appropriately set according to the viscosity to be imparted to the viscous composition according to the present invention, the content is usually preferably 0.1 to 4 parts by mass and more preferably 0.5 to 3 parts by mass relative to 100 parts by mass of water.

In the liquid composition, other than the alkyl-modified carboxyl group-containing water-soluble copolymer, the polyvalent metal salt, and water, other additives and active components may be contained according to the application of the viscous composition according to the present invention.

Although the method for producing the liquid composition is not particularly limited, examples thereof include a method of dispersing an alkyl-modified carboxyl group-containing water-soluble copolymer in water and then adding a polyvalent metal salt; a method of dissolving a polyvalent metal salt in water and then adding an alkyl-modified carboxyl group-containing water-soluble copolymer to be dispersed; a method of mixing an alkyl-modified carboxyl group-containing water-soluble copolymer and a polyvalent metal salt and then adding the mixture into water, and the like.

Although the pH of the liquid composition depends on the concentrations of the alkyl-modified carboxyl group-containing water-soluble copolymer and the polyvalent metal salt, the pH is usually about 2 to 5.

Moreover, in the liquid composition, usually, the alkyl-modified carboxyl group-containing water-soluble copolymer is present in the state of being dispersed and the polyvalent metal salt is present in the state of being dissolved. Moreover, usually, the liquid composition is not thickened with the alkyl-modified carboxyl group-containing water-soluble copolymer, and is in the state of being a dispersion liquid with low viscosity.

### Alkali treatment for the liquid composition

In the preparation of the viscous composition according to the present invention, first, a base is added to the liquid composition and the pH is adjusted to 7.0 or greater to obtain an alkaline viscous composition.

Although the base used in the pH adjustment is not particularly limited, examples thereof include sodium hydroxide, potassium hydroxide, calcium hydroxide, magnesium hydroxide, barium hydroxide, copper hydroxide, iron hydroxide, lithium hydroxide, beryllium hydroxide, triethanolamine, diisopropanolamine, 2-amino-2-methyl-1-propanol, triethylamine, ethylamine, dimethylamine, diethylamine, trimethylamine, ammonia and the like. Of these bases, potassium hydroxide, sodium hydroxide, triethanolamine, diisopropanolamine and 2-amino-2-methyl-1-propanol are inexpensive, are easily available, and are suitably used in the present invention.

These bases each may be used alone or may be used in combination of two or more kinds thereof.

Moreover, although the pH as one of the liquid properties needs only to be adjusted to 7.0 or greater by the addition of a base to the liquid composition, the pH preferably lies within the range of 7.0 to 12.0. By adjusting the pH within such a range, it is possible to impart the viscous composition according to the present invention with high viscosity.

The method for adjusting the pH of the liquid composition to 7.0 or greater with a base is not particularly limited, examples thereof include a method of adding a base to the liquid composition with stirring. The base to be added may be in a solid state or may be one made into an aqueous solution form.

By adjusting the pH of the liquid composition to 7.0 or greater in this way, usually, the alkyl-modified carboxyl group-containing water-soluble copolymer is solubilized, the liquid composition is thickened, and the alkaline viscous composition obtained is in a gel state or in a sol state. In this connection, in the present invention, the gel state refers to a state of being high in viscosity and showing semisolid state properties, and the sol state refers to a state of being high in fluidity and showing liquid state properties.

### Acid treatment for alkaline viscous composition

Then, by adding a carboxylic acid and/or a phosphorus oxo acid to the alkaline viscous composition to adjust the pH to 2.5 to 6.5, the viscous composition according to the present invention is prepared.

The carboxylic acid used in the pH adjustment is not particularly limited as long as the carboxylic acid is an acid having a carboxyl group in its molecular structure, any one of a monocarboxylic acid, a dicarboxylic acid, a tricarboxylic acid, a tetracarboxylic acid, and the like is acceptable. Specifically, examples of the carboxylic acid include acetic acid, butyric acid, lactic acid, benzoic acid, gluconic acid, oxalic acid, malonic acid, succinic acid, glutaric acid, adipic acid, phytic acid, fumaric acid, maleic acid, tartaric acid, malic acid, phthalic acid, citric acid, ethylenediaminetetraacetic acid, and the like.

Moreover, the phosphorus oxo acid used in the pH adjustment needs only to be an acid in which a hydroxyl group and an oxo group are bonded to the phosphorus atom, and examples thereof include phosphoric acid, phosphorous acid, hypophosphorous acid, diphosphoric acid, ethyl phosphate, diethyl phosphate, butyl phosphate, dibutyl phosphate, butoxyethyl phosphate, dibutoxyethyl phosphate, 2-ethylhexyl phosphate, bis-(2-ethylhexyl phosphate), isotridecyl phosphate, diisotridecyl phosphate, dodecyl phosphate, didodecyl phosphate, tetradecyl phosphate, ditetradecyl phosphate, hexadecyl phosphate, dihexadecyl phosphate, octadecyl phosphate, dioctadecyl phosphate, oleyl phosphate, dioleyl phosphate, and the like.

Of these carboxylic acids and phosphorus oxo acids, from the viewpoint of being easy to control the pH, examples thereof preferably include acetic acid, butyric acid, lactic acid, benzoic acid, gluconic acid, oxalic acid, malonic acid, succinic acid, glutaric acid, adipic acid, phytic acid, fumaric acid, maleic acid, tartaric acid, malic acid, phthalic acid, citric acid, ethylenediaminetetraacetic acid, and phosphoric acid.

These carboxylic acids and phosphorus oxo acids each may be used alone or may be used in combination of two or more kinds thereof.

Moreover, although the pH as one of the liquid properties needs only to be adjusted to 2.5 to 6.5 by the addition of a carboxylic acid and/or a phosphorus oxo acid to the alkaline viscous composition, the pH preferably lies within the range of 3.5 to 6.5. By adjusting the pH within such a range, a viscous composition imparted with high viscosity under an acidic to weakly acidic condition while containing a polyvalent metal salt is prepared.

The method for adjusting the pH of the alkaline viscous composition to 2.5 to 6.5 with a carboxylic acid and/or a phosphorus oxo acid is not particularly limited, and examples thereof include a method of adding a carboxylic acid and/or a phosphorus oxo acid to the alkaline viscous composition with stirring. The carboxylic acid and/or the phosphorus oxo acid to be added may be in a solid state or may be one made into an aqueous solution form.

By adjusting the pH of the alkaline viscous composition to 2.5 to 6.5 in this way, it is possible to allow the pH to lie in the range from acidic to weakly acidic conditions while maintaining the viscosity of the alkaline viscous composition, and the viscous composition according to the present invention is prepared.

### Properties of the viscous composition according to the present invention and applications thereof

The viscous composition according to the present invention is one that is produced by the previously mentioned production method, and the alkyl-modified carboxyl group-containing water-soluble copolymer, the polyvalent metal salt, the base, and the carboxylic acid and/or the phosphorus oxo acid are contained therein.

For example, the content of the alkyl-modified carboxyl group-containing water-soluble copolymer in the viscous composition according to the present invention is 0.1 to 4% by mass and more preferably 0.1 to 3% by mass.

Moreover, for example, the content of the polyvalent metal salt in the viscous composition according to the present invention is preferably 0.1 to 4% by mass and more preferably 0.5 to 3% by mass.

Moreover, the content of the base in the viscous composition according to the present invention is the amount required for allowing the pH as one of the liquid properties of the liquid composition to be 7.0 or greater, and although the content varies depending on the kind of the base to be used, for example, the content is preferably 0.01 to 6% by mass and more preferably 0.03 to 5% by mass.

Moreover, the content of the carboxylic acid and/or the phosphorus oxo acid in the viscous composition according to the present invention is the amount required for allowing the pH as one of the liquid properties of the alkaline viscous composition to be 2.5 to 6.5, and although the content varies depending on the kind of the carboxylic acid and/or the phosphorus oxo acid to be used, for example, the content is preferably 0.1 to 4% by mass and more preferably 0.3 to 2% by mass.

In the viscous composition according to the present invention, water constitutes the remaining portion other than the alkyl-modified carboxyl group-containing water-soluble copolymer, the polyvalent metal salt, the base, the carboxylic acid and/or the phosphorus oxo acid, and additives and active components which are added as necessary.

Other than the previously described components, the viscous composition according to the present invention may contain other additives and active components according to the application thereof. For example, in the case where the viscous composition according to the present invention is used as a cosmetic material, the viscous composition may contain a moisturizing agent, an oxidation inhibitor, a blood circulation promoter, a cold-sensing agent, an antiperspirant, a sterilization agent, a skin activator, a deodorant, a surfactant, a fragrance material, a coloring matter, and the like. In particular, in the case where the viscous composition according to the present invention is used as a cosmetic material, from the viewpoints of eliminating the stickiness to the skin and further enhancing the satisfactory feeling of use which gives a smooth dry feeling, it is desirable that, for example, a moisturizing agent composed of glycerin, propylene glycol, dipropylene glycol, 1,3-butylene glycol, polyethylene glycol, sorbitol, sodium lactate, sodium 2-pyrrolidone-5-carboxylate, sodium hyaluronate, acetylated sodium hyaluronate, or the like be blended therein.

In the case where other additives and active components are contained in the viscous composition according to the present invention, these additives and active components may be added to the liquid composition before the alkali treatment of the liquid composition or these additives and active components may be added after the acid treatment of the alkaline viscous composition.

For example, the degree of viscosity of the viscous composition according to the present invention is determined to be 1,000 to 100,000 mPa•s at 25°C. The degree of viscosity is preferably 1,000 to 60,000 mPa•s, further preferably 1,000 to 15,000 mPa•s, and still further preferably 1,000 to 10,000 mPa•s. The viscous composition according to the present invention can be provided with high viscosity in this way even while containing a polyvalent metal salt under an acidic to weakly acidic condition.

In this connection, the viscosity is determined by measuring the viscosity at 25°C at the end of 1 minute after the initiation of rotation using a BH type rotary viscosity meter (available from SHIBAURA SEMTEK CO., LTD., a single cylinder type rotary viscosity meter (VISMETRON), model: VS-11H) while the rotating speed is set to 20 rotations per minute. As the rotor used for the measurement, a rotor No. 3 in the case where the sample has a viscosity lower than 2,000 mPa•s, a rotor No. 4 in the case where the sample has a viscosity higher than or equal to 2,000 mPa•s and lower than 5,000 mPa•s, a rotor No. 5 in the case where the sample has a viscosity higher than or equal to 5,000 mPa•s and lower than 15,000 mPa•s, a rotor No. 6 in the case where the sample has a viscosity higher than or equal to 15,000 mPa•s and lower than 40,000 mPa•s, or a rotor No. 7 in the case where the sample has a viscosity higher than or equal to 40,000 mPa•s is employed.

The viscous composition according to the present invention is imparted with the above-mentioned viscosity, and exhibits a state such as a viscous liquid state, a fluid gel-like state and a cream-like state.

The viscous composition according to the present invention is used in product categories such as a cosmetic material, medical and pharmaceutical products (in particular, an external preparation for skin), a toiletry product, a household product and a water-soluble coating material. Above all, since the viscous composition according to the present invention suppresses the feeling of stickiness even when applied to a skin as well as has a high viscosity and a feeling of use which gives a smooth dry feeling and a feeling of freshness is attained, the viscous composition is suitably used as a cosmetic material, an external preparation for skin, or a toiletry product.

In the case where the viscous composition according to the present invention constitutes a cosmetic material, although the form of the formulation is not particularly limited, examples thereof include a skin lotion, a milky lotion, a beauty liquid, a cream, a cream pack material, a massage cream, a hair setting gel, a sunscreen, a styling gel, an eyeliner, mascara, a lipstick, foundation and the like. Moreover, in the case where the viscous composition according to the present invention constitutes a toiletry product, although the form of the formulation is not particularly limited, examples thereof include a cleansing cream, a cleansing gel, face-washing foam, a hair wash, a body wash, a rinse and the like.

Moreover, in the case where the viscous composition according to the present invention constitutes a cosmetic material, when a polyvalent metal salt of an ascorbic acid derivative such as magnesium ascorbyl phosphate, magnesium sulfo-L-ascorbate, calcium ascorbyl phosphate and calcium sulfo-L-ascorbate is used as the polyvalent metal salt, based on the skin whitening action which these polyvalent metal salts have, the viscous composition according to the present invention can be suitably used as a cosmetic material for skin whitening.

### EXAMPLES

The present invention will be described below in detail with reference to examples and comparative examples, but the present invention should not be limited by these examples at all.

### 1. Preparation of viscous composition and evaluation thereof

### [Measuring method]

Viscous compositions obtained in respective examples and comparative examples were evaluated for the viscosity and the texture sensory test in the following manner.

### (1) Degree of viscosity

Each evaluation sample was measured for the degree of viscosity at 25°C at the end of 1 minute using a BH type rotary viscosity meter (available from SHIBAURA SEMTEK CO., LTD., a single cylinder type rotary viscosity meter (VISMETRON), model: VS-11H) while the rotating speed was set to 20 rotations per minute. As the rotor used for the measurement, a rotor No. 3 in the case where the sample had a viscosity lower than 2,000 mPa•s, a rotor No. 4 in the case where the sample had a viscosity higher than or equal to 2,000 mPa•s and lower than 5,000 mPa•s, a rotor No. 5 in the case where the sample had a viscosity higher than or equal to 5,000 mPa•s and lower than 15,000 mPa•s, a rotor No. 6 in the case where the sample had a viscosity higher than or equal to 15,000 mPa•s and lower than 40,000 mPa•s, or a rotor No. 7 in the case where the sample had a viscosity higher than or equal to 40,000 mPa•s was employed. In this connection, since the sample with a viscosity higher than or equal to 1,000 mPa•s can be provided with characteristics of hardly dribbling at the time of application, the sample can be judged to be a satisfactory sample having a high viscosity.

### (2) Feeling of use

A proper amount of each of viscous compositions obtained in respective examples and comparative examples was put on the skin and applied by spreading the composition with the fingers. The feeling of use after applied was scored according to the following criteria. In this connection, the evaluation for the feeling of use was performed by calculating the total marks determined by 10 panelists (males and females of 5 respondents respectively). In the case where the total number of points as marks is greater than or equal to 25 points, the composition can be judged to have a satisfactory feeling of use that can satisfy the requirement on the fresh tactile feeling at a practical level.

### <Criteria for the feeling of use>

4 Points: A smooth dry tactile feeling and a feeling of freshness.
3 Points: A smooth dry tactile feeling and a slight feeling of freshness.
2 Points: No feeling of stickiness but no feeling of freshness.
1 Point: A feeling of stickiness.

### [Production Example 1]

In a 500-mL four-necked flask equipped with a stirrer, a thermometer, a nitrogen inlet tube and a condenser, 45 g (0.625 mole) of acrylic acid, 0.45 g of BLEMMER VMA70 (available from NOF CORPORATION: the mixture of 10 to 20 parts by mass of stearyl methacrylate, 10 to 20 parts by mass of eicosanyl methacrylate, 59 to 80 parts by mass of behenyl methacrylate and 1 part by mass or less of tetracosanyl methacrylate) as a (meth)acrylic acid alkyl ester having an alkyl group with 18 to 24 carbon atoms, 150 g of normal hexane, and 0.081 g (0.00035 mole) of 2,2'-azobismethylisobutylate were placed. Subsequently, the contents were stirred and uniformly mixed, after which nitrogen gas was blown into the solution in order to remove oxygen existing in the upper space part of the reaction vessel, the raw materials and the solvent. Then, under a nitrogen atmosphere, the contents were allowed to undergo a reaction for 4 hours while maintaining the temperature at 60 to 65°C. After the completion of the reaction, the slurry produced was heated to 90°C to remove normal hexane, and furthermore, the contents were dried under reduced pressure for 8 hours at 110°C at 10 mmHg to obtain 43 g of a finely powdered white alkyl-modified carboxyl group-containing water-soluble copolymer.

### [Example 1]

In a 200-mL plastic beaker, 100 g of distilled water was placed, and 1.0 g of the alkyl-modified carboxyl group-containing water-soluble copolymer obtained in Production Example 1 was added, stirred for 30 minutes at 5,000 rpm using a stirrer (available from PRIMIX Corporation, model: T.K. ROBOMIX, the dispering impeller with a blade diameter of 30 mm), and uniformly dispersed. Next, 2.0 g of magnesium ascorbyl phosphate was added, stirred for 5 minutes at 2,000 rpm using a stirrer, and uniformly dispersed to obtain a liquid composition. The liquid composition was measured for the pH at this point using a pH meter (available from HORIBA, Ltd., model: D-51), whereupon the pH was determined to be 4.5.

To the obtained liquid composition, 3.0 g of an aqueous 18% by mass sodium hydroxide solution was added, stirred for 5 minutes at 2,000 rpm using a stirrer, and uniformly dispersed to obtain an alkaline viscous composition. At this point, the pH of the alkaline viscous composition was determined to be 11.2.

Next, to the alkaline viscous composition, 0.9 g of an aqueous 50% by mass citric acid solution was added, and by stirring the contents for 5 minutes at 2,000 rpm using a stirrer and allowing the contents to be uniformly dispersed, a viscous composition was finally obtained. The pH of the obtained viscous composition was determined to be 6.0.

### [Example 2]

A viscous composition was obtained in the same manner as that in Example 1 except that the amount of the alkyl-modified carboxyl group-containing water-soluble copolymer was changed to 2.0 g, the amount of the aqueous 18% by mass sodium hydroxide solution was changed to 6.0 g, and the amount of the aqueous 50% by mass citric acid solution was changed to 1.8 g, in Example 1. In this connection, the pH of the liquid composition obtained before the addition of sodium hydroxide was determined to be 4.2, the pH of the alkaline viscous composition obtained after the addition of sodium hydroxide was determined to be 11.4, and the pH of the finally obtained viscous composition was determined to be 6.0.

### [Example 3]

A viscous composition was obtained in the same manner as that in Example 1 except that the amount of the alkyl-modified carboxyl group-containing water-soluble copolymer was changed to 3.0 g, the amount of the aqueous 18% by mass sodium hydroxide solution was changed to 9.0 g, and the amount of the aqueous 50% by mass citric acid solution was changed to 2.3 g, in Example 1. In this connection, the pH of the liquid composition obtained before the addition of sodium hydroxide was determined to be 4.0, the pH of the alkaline viscous composition obtained after the addition of sodium hydroxide was determined to be 11.5, and the pH of the finally obtained viscous composition was determined to be 6.0.

### [Example 4]

A viscous composition was obtained in the same manner as that in Example 1 except that the amount of the aqueous 50% by mass citric acid solution was changed to 1.6 g, in Example 1. In this connection, the pH of the liquid composition obtained before the addition of sodium hydroxide was determined to be 4.5, the pH of the alkaline viscous composition obtained after the addition of sodium hydroxide was determined to be 10.8, and the pH of the finally obtained viscous composition was determined to be 5.0.

### [Example 5]

A viscous composition was obtained in the same manner as that in Example 1 except that the amount of the aqueous 50% by mass citric acid solution was changed to 2.2 g, in Example 1. In this connection, the pH of the liquid composition obtained before the addition of sodium hydroxide was determined to be 4.4, the pH of the alkaline viscous composition obtained after the addition of sodium hydroxide was determined to be 11.2, and the pH of the finally obtained viscous composition was determined to be 4.0.

### [Example 6]

A viscous composition was obtained in the same manner as that in Example 1 except that 2.0 g of the magnesium ascorbyl phosphate was changed to 2.0 g of magnesium chloride, in Example 1. In this connection, the pH of the liquid composition obtained before the addition of sodium hydroxide was determined to be 2.5, the pH of the alkaline viscous composition obtained after the addition of sodium hydroxide was determined to be 11.0, and the pH of the finally obtained viscous composition was determined to be 6.0.

### [Example 7]

A viscous composition was obtained in the same manner as that in Example 1 except that 0.9 g of the aqueous 50% by mass citric acid solution was changed to 0.8 g of an aqueous 50% by mass lactic acid solution, in Example 1. In this connection, the pH of the liquid composition obtained before the addition of sodium hydroxide was determined to be 4.5, the pH of the alkaline viscous composition obtained after the addition of sodium hydroxide was determined to be 11.2, and the pH of the finally obtained viscous composition was determined to be 6.0.

### [Example 8]

A viscous composition was obtained in the same manner as that in Example 1 except that 0.9 g of the aqueous 50% by mass citric acid solution was changed to 0.7 g of an aqueous 50% by mass ethylenediaminetetraacetic acid (EDTA) solution, in Example 1. In this connection, the pH of the liquid composition obtained before the addition of sodium hydroxide was determined to be 4.3, the pH of the alkaline viscous composition obtained after the addition of sodium hydroxide was determined to be 10.9, and the pH of the finally obtained viscous composition was determined to be 6.0.

### [Example 9]

A viscous composition was obtained in the same manner as that in Example 1 except that 0.9 g of the aqueous 50% by mass citric acid solution was changed to 3.0 g of an aqueous 20% by mass phosphoric acid solution, in Example 1. In this connection, the pH of the liquid composition obtained before the addition of sodium hydroxide was determined to be 4.4, the pH of the alkaline viscous composition obtained after the addition of sodium hydroxide was determined to be 10.9, and the pH of the finally obtained viscous composition was determined to be 6.0.

### [Example 10]

A viscous composition was obtained in the same manner as that in Example 1 except that the amount of the magnesium ascorbyl phosphate was changed to 0.5 g, and the amount of the aqueous 50% by mass citric acid solution was changed to 0.8 g, in Example 1. In this connection, the pH of the liquid composition obtained before the addition of sodium hydroxide was determined to be 3.9, the pH of the alkaline viscous composition obtained after the addition of sodium hydroxide was determined to be 11.0, and the pH of the finally obtained viscous composition was determined to be 6.0.

### [Example 11]

A viscous composition was obtained in the same manner as that in Example 1 except that the amount of the magnesium ascorbyl phosphate was changed to 3.0 g, and the amount of the aqueous 50% by mass citric acid solution was changed to 1.1 g, in Example 1. In this connection, the pH of the liquid composition obtained before the addition of sodium hydroxide was determined to be 4.6, the pH of the alkaline viscous composition obtained after the addition of sodium hydroxide was determined to be 10.8, and the pH of the finally obtained viscous composition was determined to be 6.0.

### [Example 12]

A viscous composition was obtained in the same manner as that in Example 1 except that 100 g of the distilled water was changed to 100 g of distilled water and 8.0 g of glycerin, in Example 1. In this connection, the pH of the liquid composition obtained before the addition of sodium hydroxide was determined to be 4.5, the pH of the alkaline viscous composition obtained after the addition of sodium hydroxide was determined to be 11.3, and the pH of the finally obtained viscous composition was determined to be 6.0.

### [Example 13]

A viscous composition was obtained in the same manner as that in Example 1 except that 100 g of the distilled water was changed to 100 g of distilled water and 8.0 g of 1,3-butanediol, in Example 1. In this connection, the pH of the liquid composition obtained before the addition of sodium hydroxide was determined to be 4.3, the pH of the alkaline viscous composition obtained after the addition of sodium hydroxide was determined to be 10.9, and the pH of the finally obtained viscous composition was determined to be 6.0.

### [Comparative Example 1]

In a 200-mL plastic beaker, 100 g of distilled water was placed, and 1.0 g of the alkyl-modified carboxyl group-containing water-soluble copolymer obtained in Production Example 1 was added, stirred for 30 minutes at 5,000 rpm using a stirrer (available from PRIMIX Corporation, model: T.K. ROBOMIX, the dispering impeller with a blade diameter of 30 mm), and uniformly dispersed. Next, 2.0 g of magnesium ascorbyl phosphate was added, stirred for 5 minutes at 2,000 rpm using a stirrer, and uniformly dispersed to obtain a liquid composition. At this point, the pH of the liquid composition was determined to be 4.5.

To the obtained liquid composition, 2.5 g of an aqueous 18% by mass sodium hydroxide solution was added, stirred for 5 minutes at 2,000 rpm using a stirrer, and uniformly dispersed to obtain a viscous composition. At this point, the pH of the viscous composition was determined to be 6.0.

### [Comparative Example 2]

In a 200-mL plastic beaker, 100 g of distilled water was placed, and 1.0 g of the alkyl-modified carboxyl group-containing water-soluble copolymer obtained in Production Example 1 was added, stirred for 30 minutes at 5,000 rpm using a stirrer (available from PRIMIX Corporation, model: T.K. ROBOMIX, the dispering impeller with a blade diameter of 30 mm), and uniformly dispersed. Next, 2.0 g of magnesium ascorbyl phosphate was added, stirred for 5 minutes at 2,000 rpm using a stirrer, and uniformly dispersed to obtain a liquid composition. At this point, the pH of the liquid composition was determined to be 4.5.

To the obtained liquid composition, 0.9 g of an aqueous 50% by mass citric acid solution was added, stirred for 5 minutes at 2,000 rpm using a stirrer, and uniformly dispersed. At this point, the pH was determined to be 3.6.

Next, 3.0 g of an aqueous 18% by mass sodium hydroxide solution was added, stirred for 5 minutes at 2,000 rpm using a stirrer, and uniformly dispersed to obtain a viscous composition. At this point, the pH of the viscous composition was determined to be 6.0.

### [Comparative Example 3]

A viscous composition was obtained in the same manner as that in Example 1 except that 0.9 g of the aqueous 50% by mass citric acid solution was changed to 6.8 g of 1 mol/L hydrochloric acid, in Example 1. In this connection, the pH of the liquid composition obtained before the addition of sodium hydroxide was determined to be 4.4, the pH of the alkaline viscous composition obtained after the addition of sodium hydroxide was determined to be 10.9, and the pH of the finally obtained viscous composition was determined to be 6.0.

### [Reference Example 1]

In a 200-mL plastic beaker, 100 g of distilled water was placed, and 1.0 g of the alkyl-modified carboxyl group-containing water-soluble copolymer obtained in Production Example 1 was added, stirred for 30 minutes at 5,000 rpm using a stirrer (available from PRIMIX Corporation, model: T.K. ROBOMIX, the dispering impeller with a blade diameter of 30 mm), and uniformly dispersed. Next, 2.0 g of sodium ascorbate was added, stirred for 5 minutes at 2,000 rpm using a stirrer, and uniformly dispersed to obtain a liquid composition. At this point, the pH of the liquid composition was determined to be 4.5.

To the obtained liquid composition, 2.0 g of an aqueous 18% by mass sodium hydroxide solution was added, stirred for 5 minutes at 2,000 rpm using a stirrer, and uniformly dispersed to obtain a viscous composition. At this point, the pH of the viscous composition was determined to be 6.0.

### [Summary of evaluation results]

The viscous compositions obtained in respective Examples, Comparative Examples and Reference Example were evaluated for the degree of viscosity and the feeling of use, and the results are shown in Table 1. As apparent from Table 1, by adjusting the pH of a liquid composition containing an alkyl-modified carboxyl group-containing water-soluble copolymer obtained in Production Example 1, a polyvalent metal salt and water to 7.0 or greater with a base, and then, adjusting the pH to 6.5 or less with a carboxylic acid or a phosphorus oxo acid, viscous compositions imparted with high viscosity have been obtained (Examples 1 to 13). On the other hand, in the case of adjusting the pH of a liquid composition directly to 6.0 with a base (Comparative Example 1) and the case of adding a carboxylic acid to a liquid composition and then adding a base to the liquid composition to adjust the pH to 6.0 (Comparative Example 2), the viscosity has failed to be increased to a high level thereof. Furthermore, even in the case of adjusting the pH of a liquid composition to 7.0 or greater with a base and then to 6.5 or less with hydrochloric acid, a viscous composition with high viscosity has failed to be obtained (Comparative Example 3).

In this connection, as shown in Reference Example 1, in the case of allowing an alkyl-modified carboxyl group-containing water-soluble copolymer obtained in Production Example 1 and a monovalent metal salt (sodium ascorbate) to coexist, just by simply adjusting the pH directly to 6.0 with a base, the viscosity can be increased to a high level thereof. Thus, it has also been confirmed that failing to be increased to a high level of the viscosity over a pH range from acidic to weakly acidic conditions in the coexistence of a polyvalent metal salt is a drawback peculiar to the alkyl-modified carboxyl group-containing water-soluble copolymer obtained in Production Example 1.

Moreover, it has also been confirmed that the feeling of use is satisfactory since the viscous compositions obtained in respective Examples give no feeling of stickiness even when applied to a skin and give a smooth dry feeling, and the skin is imparted with freshness.

**[Table 1]**

| | Degree of viscosity [mPa•s] | Feeling of use [score of the total number of points as marks] |
|---|---|---|
| Example 1 | 4,200 | 34 |
| Example 2 | 50,000 | 30 |
| Example 3 | 85,000 | 25 |
| Example 4 | 5,200 | 34 |
| Example 5 | 2,200 | 36 |
| Example 6 | 5,500 | 34 |
| Example 7 | 3,300 | 35 |
| Example 8 | 4,000 | 35 |
| Example 9 | 2,500 | 36 |
| Example 10 | 9,200 | 33 |
| Example 11 | 1,100 | 38 |
| Example 12 | 2,000 | 39 |
| Example 13 | 6,500 | 38 |
| Comparative Example 1 | 20 | 37 |
| Comparative Example 2 | 800 | 35 |
| Comparative Example 3 | 50 | 37 |
| Reference Example 1 | 6,000 | 34 |

### [Example 14]

In a 200-mL plastic beaker, 100 g of distilled water was placed, and 1.0 g of the alkyl-modified carboxyl group-containing water-soluble copolymer obtained in Production Example 1 was added, stirred for 30 minutes at 5,000 rpm using a stirrer (available from PRIMIX Corporation, model: T.K. ROBOMIX, the dispering impeller with a blade diameter of 30 mm), and uniformly dispersed. Next, 2.0 g of magnesium ascorbyl phosphate was added, stirred for 5 minutes at 2,000 rpm using a stirrer, and uniformly dispersed to obtain a liquid composition. The liquid composition was measured for the pH at this point, whereupon the pH was determined to be 4.5.

While the obtained liquid composition was stirred at 2,000 rpm with a stirrer, an aqueous 18% by mass sodium hydroxide solution was gradually added dropwise until the pH reached 11.2 to obtain an alkaline viscous composition. During the period of time until the pH reached 11.2, the measurement for the degree of viscosity was also performed.

Next, while the obtained alkaline viscous composition (pH 11.2) was stirred at 2,000 rpm with a stirrer, an aqueous 50% by mass citric acid solution was gradually added dropwise until the pH reached 5.0 to obtain a viscous composition. During the period of time until the pH reached 5.0, the measurement for the degree of viscosity was also performed.

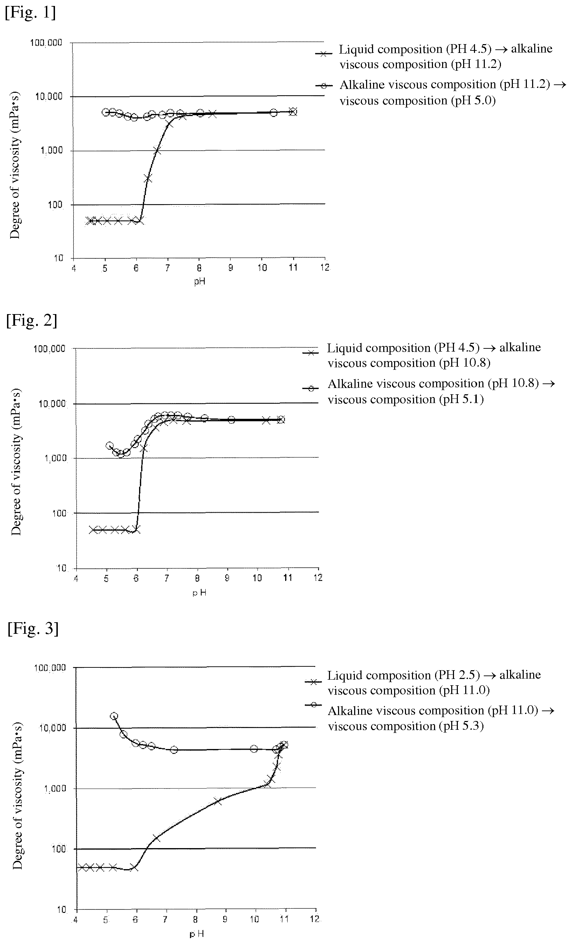

The results obtained by performing the measurement for the degree of viscosity during the period of time until an alkaline viscous composition (pH 11.2) was obtained from a liquid composition (PH 4.5) and the period of time until a viscous composition (pH 5.0) was obtained from the alkaline viscous composition (pH 11.2) are shown in Fig. 1. From these results, it has been confirmed that the degree of viscosity rises as the pH of the liquid composition (PH 4.5) is raised, and then, the degree of viscosity equivalent to that of the alkaline viscous composition (pH 11.2) is maintained even when the pH is lowered by using the citric acid.

### [Example 15]

In a 200-mL plastic beaker, 100 g of distilled water was placed, and 1.0 g of the alkyl-modified carboxyl group-containing water-soluble copolymer obtained in Production Example 1 was added, stirred for 30 minutes at 5,000 rpm using a stirrer (available from PRIMIX Corporation, model: T.K. ROBOMIX, the dispering impeller with a blade diameter of 30 mm), and uniformly dispersed. Next, 2.0 g of magnesium ascorbyl phosphate was added, stirred for 5 minutes at 2,000 rpm using a stirrer, and uniformly dispersed to obtain a liquid composition. The liquid composition was measured for the pH at this point, whereupon the pH was determined to be 4.5.

While the obtained liquid composition was stirred at 2,000 rpm with a stirrer, an aqueous 18% by mass sodium hydroxide solution was gradually added dropwise until the pH reached 10.8 to obtain an alkaline viscous composition. During the period of time until the pH reached 10.8, the measurement for the degree of viscosity was also performed.

Next, while the obtained alkaline viscous composition (pH 10.8) was stirred at 2,000 rpm with a stirrer, an aqueous 50% by mass lactic acid solution was gradually added dropwise until the pH reached 5.1 to obtain a viscous composition. During the period of time until the pH reached 5.1, the measurement for the degree of viscosity was also performed.

The results obtained by performing the measurement for the degree of viscosity during the period of time until an alkaline viscous composition (pH 10.8) was obtained from a liquid composition (PH 4.2) and the period of time until a viscous composition (pH 5.1) was obtained from the alkaline viscous composition (pH 10.8) are shown in Fig. 2. From these results, as in the case of Example 14, it has been found that the degree of viscosity rises as the pH of the liquid composition (PH 4.2) is raised, and then, the degree of viscosity can be maintained high even when the pH is lowered by using the lactic acid.

### [Example 16]

In a 200-mL plastic beaker, 100 g of distilled water was placed, and 1.0 g of the alkyl-modified carboxyl group-containing water-soluble copolymer obtained in Production Example 1 was added, stirred for 30 minutes at 5,000 rpm using a stirrer (available from PRIMIX Corporation, model: T.K. ROBOMIX, the dispering impeller with a blade diameter of 30 mm), and uniformly dispersed. Next, 2.0 g of magnesium chloride was added, stirred for 5 minutes at 2,000 rpm using a stirrer, and uniformly dispersed to obtain a liquid composition. The liquid composition was measured for the pH at this point, whereupon the pH was determined to be 2.5.

While the obtained liquid composition was stirred at 2,000 rpm with a stirrer, an aqueous 18% by mass sodium hydroxide solution was gradually added dropwise until the pH reached 11.0 to obtain an alkaline viscous composition. During the period of time until the pH reached 11.0, the measurement for the degree of viscosity was also performed.

Next, while the obtained alkaline viscous composition (pH 11.0) was stirred at 2,000 rpm with a stirrer, an aqueous 50% by mass citric acid solution was gradually added dropwise until the pH reached 5.3 to obtain a viscous composition. During the period of time until the pH reached 5.3, the measurement for the degree of viscosity was also performed.

The results obtained by performing the measurement for the degree of viscosity during the period of time until an alkaline viscous composition (pH 11.0) was obtained from a liquid composition (PH 2.5) and the period of time until a viscous composition (pH 5.3) was obtained from the alkaline viscous composition (pH 11.0) are shown in Fig. 3. From these results, even in the case where magnesium chloride is used as a polyvalent metal salt, it has been found that the degree of viscosity rises as the pH of the liquid composition (PH 2.5) is raised, and then, the degree of viscosity can be maintained high even when the pH is lowered by using the citric acid.

### 2. Formulation Examples

### [Formulation Example 1] Whitening skin lotion

Ingredients 1 to 9 shown in Table 2 were uniformly mixed and dispersed, after which an ingredient 10 was added and mixed therewith, and then, by adding ingredients 11 and 12 thereto and uniformly stirring, a whitening skin lotion was prepared.

**[Table 2]**

| (Ingredients) | | (% by mass) |
|---|---|---|
| 1. | Alkyl-modified carboxyl group-containing water-soluble copolymer obtained in Production Example 1 | 1.0 |
| 2. | Purified water | 50.0 |
| 3. | Pentylene glycol | 1.2 |
| 4. | Phenoxyethanol | 0.6 |
| 5. | Glycerin | 4.0 |
| 6. | Magnesium ascorbyl phosphate | 2.5 |
| 7. | Gardenia jasminoides extract | 0.3 |
| 8. | Sophora flavescens extract | 0.3 |
| 9. | Raffinose | 0.5 |
| 10. | Aqueous 18% sodium hydroxide solution | 3.0 |
| 11. | Citric acid | Proper quantity |
| 12. | Purified water | Residual quantity |
| | Total | 100 |
| | pH | 5.80 |

### [Formulation Example 2] Skin care lotion

Ingredients 1 to 3 shown in Table 3 were mixed to prepare a first mixture. Moreover, separately, ingredients 4 to 7 were mixed to prepare a second mixture. The first mixture was added dropwise to the second mixture in a state of being heated at 80°C and stirred to be emulsified. Then, an ingredient 8 was added dropwise thereto and stirring was performed, after which an ingredient 9 was added dropwise thereto and stirring was performed. Furthermore, an ingredient 10 was added thereto and stirring was performed, and by cooling, a skin care lotion was prepared.

**[Table 3]**

| (Ingredients) | | (% by mass) |
|---|---|---|
| 1. | Alkyl-modified carboxyl group-containing water-soluble copolymer obtained in Production Example 1 | 0.2 |
| 2. | Dicaprylic acid triglyceride | 6.3 |
| 3. | 2-ethylhexyl stearate | 7.1 |
| 4. | Purified water | 70.0 |
| 5. | Glycerin | 2.9 |
| 6. | Magnesium ascorbyl phosphate | 2.0 |
| 7. | Polysorbate 60 | 1.9 |
| 8. | Aqueous 6% sodium hydroxide solution | 1.1 |
| 9. | Lactic acid | Proper quantity |
| 10. | Purified water | Residual quantity |
| | Total | 100 |
| | pH | 6.0 |

### [Formulation Example 3] Skin whitening component-formulated moisturizing cream

Ingredients 1 to 4 shown in Table 4 were mixed to prepare a first mixture. Moreover, separately, ingredients 5 to 8 were mixed to prepare a second mixture. The first mixture was added dropwise to the second mixture in a state of being heated at 75°C and stirred to be emulsified. Then, an ingredient 9 was added dropwise thereto and stirring was performed, after which an ingredient 10 was added dropwise thereto and stirring was performed. Furthermore, an ingredient 11 was added thereto and stirring was performed to allow the contents to cool. At the point of time when the temperature was lowered to room temperature, by further adding an ingredient 12 dropwise thereto and stirring, a skin whitening component-formulated moisturizing cream was prepared.

**[Table 4]**

| (Ingredients) | | (% by mass) |
|---|---|---|
| 1. | Alkyl-modified carboxyl group-containing water-soluble copolymer obtained in Production Example 1 | 0.2 |
| 2. | Purified water | 60.0 |
| 3. | Glycerin | 5.0 |
| 4. | Polysorbate 60 | 1.0 |
| 5. | Magnesium ascorbyl phosphate | 4.0 |
| 6. | Cetyl alcohol | 1.0 |
| 7. | Dicaprylic acid triglyceride | 10.3 |
| 8. | Isopropyl myristate | 4.0 |
| 9. | Aqueous 6% NaOH solution | 1.1 |
| 10. | Citric acid | Proper quantity |
| 11. | Purified water | Residual quantity |
| 12. | Ethanol | 3.0 |
| | pH | 5.5 |

### [Formulation Example 4] Cleansing gel

Ingredients 1 to 3 shown in Table 5 were mixed to prepare a first mixture. Moreover, separately, ingredients 4 to 6 were mixed to prepare a second mixture. Then, the first mixture and the second mixture were stirred to be mixed, and to this, a mixture of an ingredient 7 and an ingredient 8 was added and mixed. Afterward, an ingredient 9 was added dropwise thereto and stirring was performed. Then, by adding a mixture of an ingredient 10 and an ingredient 11 dropwise thereto and performing stirring, a cleansing gel was prepared.

**[Table 5]**

| (Ingredients) | | (% by mass) |
|---|---|---|
| 1. | Sodium laureth sulfate | 4.3 |
| 2. | Glycerin | 0.5 |
| 3. | Cocamidopropyl betaine | 3.2 |
| 4. | Purified water | 70.0 |
| 5. | Alkyl-modified carboxyl group-containing water-soluble copolymer obtained in Production Example 1 | 1.1 |
| 6. | Xanthan gum | 0.1 |
| 7. | Calcium chloride | 10.3 |
| 8. | Purified water | 4.0 |
| 9. | Aqueous 18% NaOH solution | 3.0 |
| 10. | Citric acid | Proper quantity |
| 11. | Purified water | Residual quantity |
| | pH | 5.6 |

The cosmetic materials obtained in Formulation Examples 1 to 4 each have a high viscosity and give no feeling of stickiness, and a satisfactory feeling of use which gives a feeling of freshness has been achieved.

## Claims

1. A viscous composition, being obtained by adjusting the pH of a liquid composition containing an alkyl-modified carboxyl group-containing water-soluble copolymer obtained by allowing 100 parts by mass of a (meth)acrylic acid, 0.5 to 5 parts by mass of a (meth)acrylic acid alkyl ester having an alkyl group with 18 to 24 carbon atoms and 0 to 0.1 part by mass of a compound having two or more ethylenically unsaturated groups to undergo a polymerization, a polyvalent metal salt and water to 7.0 or greater with a base, and then, adjusting the pH to 2.5 to 6.5 with at least one kind of acid selected from the group consisting of a carboxylic acid and a phosphorus oxo acid, wherein the content of the alkyl-modified carboxyl group-containing water-soluble copolymer in the liquid composition is 0.1 to 4 parts by mass relative to 100 parts by mass of water.

2. The viscous composition according to claim 1, wherein the polyvalent metal salt is a polyvalent metal salt of an organic acid.

3. The viscous composition according to claim 1 or 2, wherein the polyvalent metal salt is a polyvalent metal salt of an ascorbic acid derivative.

4. The viscous composition according to any one of claims 1 to 3, wherein the content of the alkyl-modified carboxyl group-containing water-soluble copolymer in the liquid composition is 0.1 to 3 parts by mass relative to 100 parts by mass of water.

5. The viscous composition according to any one of claims 1 to 4, wherein the content of the polyvalent metal salt in the liquid composition is 0.1 to 4 parts by mass relative to 100 parts by mass of water.

6. The viscous composition according to any one of claims 1 to 5, being a cosmetic material, an external preparation for skin, or a toiletry product.

7. A viscous composition, comprising (A) an alkyl-modified carboxyl group-containing water-soluble copolymer obtained by allowing 100 parts by mass of a (meth)acrylic acid, 0.5 to 5 parts by mass of a (meth)acrylic acid alkyl ester having an alkyl group with 18 to 24 carbon atoms and 0 to 0.1 part by mass of a compound having two or more ethylenically unsaturated groups to undergo a polymerization, (B) a polyvalent metal salt, (C) a base, (D) at least one kind of acid selected from the group consisting of a carboxylic acid and a phosphorus oxo acid, and (E) water, wherein the content of the alkyl-modified carboxyl group-containing water-soluble copolymer is 0.1 to 4 parts by mass relative to 100 parts by mass of water,
the pH being 2.5 to 6.5, and
the degree of viscosity at 25°C being 1,000 to 100,000 mPa•s.

8. The viscous composition according to claim 7, wherein the polyvalent metal salt is a polyvalent metal salt of an organic acid.

9. The viscous composition according to claim 7 or 8, wherein the alkyl-modified carboxyl group-containing water-soluble copolymer, the polyvalent metal salt, the base, and the carboxylic acid and/or the phosphorus oxo acid are contained at respective contents of 0.1 to 4% by mass, 0.1 to 4% by mass, 0.01 to 6% by mass, and 0.1 to 4% by mass.

10. The viscous composition according to any one of claims 7 to 9, being a cosmetic material, an external preparation for skin, or a toiletry product.

11. A method for producing a viscous composition, comprising the following first to third steps of:
a first step for preparing a liquid composition containing an alkyl-modified carboxyl group-containing water-soluble copolymer obtained by allowing 100 parts by mass of a (meth)acrylic acid, 0.5 to 5 parts by mass of a (meth)acrylic acid alkyl ester having an alkyl group with 18 to 24 carbon atoms and 0 to 0.1 part by mass of a compound having two or more ethylenically unsaturated groups to undergo a polymerization, a polyvalent metal salt and water,
wherein the content of the alkyl-modified carboxyl group-containing water-soluble copolymer in the liquid composition is 0.1 to 4 parts by mass relative to 100 parts by mass of water,
a second step for preparing an alkaline viscous composition by adding a base to the liquid composition to adjust the pH to 7.0 or greater, and
a third step for preparing a viscous composition by adding at least one kind of acid selected from the group consisting of a carboxylic acid and a phosphorus oxo acid to the alkaline viscous composition to adjust the pH to 2.5 to 6.5.

12. The method for producing a viscous composition according to claim 11, wherein the polyvalent metal salt is a polyvalent metal salt of an organic acid.

13. The method for producing a viscous composition according to claim 11 or 12, wherein the polyvalent metal salt is a polyvalent metal salt of an ascorbic acid derivative.

14. The method for producing a viscous composition according to any one of claims 11 to 13, wherein the content of the alkyl-modified carboxyl group-containing water-soluble copolymer in the liquid composition is 0.1 to 3 parts by mass relative to 100 parts by mass of water.

15. The method for producing a viscous composition according to any one of claims 11 to 14, wherein the content of the polyvalent metal salt in the liquid composition is 0.1 to 4 parts by mass relative to 100 parts by mass of water.

## Patentansprüche

1. Viskose Zusammensetzung, die durch Einstellen des pH einer flüssigen Zusammensetzung, enthaltend ein Alkyl-modifiziertes Carboxylgruppen-hältiges wasserlösliches Copolymer, das erhalten wurde, indem 100 Gewichtsteile einer (Meth)acrylsäure, 0,5 bis 5 Gewichtsteile eines (Meth)acrylsäurealkylesters, der eine Alkylgruppe mit 18 bis 24 Kohlenstoffatomen aufweist, und 0 bis 0,1 Gewichtsteile einer Verbindung, die zwei oder mehr ethylenisch ungesättigte Gruppen aufweist, einer Polymerisierung unterzogen wurden, ein polyvalentes Metallsalz und Wasser, auf 7,0 oder mehr mit einer Base und dann das Einstellen des pH auf 2,5 bis 6,5 mit zumindest einer Art von Säure, die aus der aus Carbonsäure und Phosphoroxosäure bestehenden Gruppe ausgewählt ist, erhalten wird, wobei der Gehalt des Alkyl-modifizierten Carboxylgruppen-hältigen wasserlöslichen Copolymers in der flüssigen Zusammensetzung 0,1 bis 4 Gewichtsteile bezogen auf 100 Gewichtsteile von Wasser beträgt.

2. Viskose Zusammensetzung nach Anspruch 1, wobei das polyvalente Metallsalz ein polyvalentes Metallsalz einer organischen Säure ist.

3. Viskose Zusammensetzung nach Anspruch 1 bis 2, wobei das polyvalente Metallsalz ein polyvalentes Metallsalz eines Ascorbinsäurederivats ist.

4. Viskose Zusammensetzung nach einem der Ansprüche 1 bis 3, wobei der Gehalt des Alkyl-modifizierten Carboxylgruppen-hältigen wasserlöslichen Copolymers in der flüssigen Zusammensetzung 0,1 bis 3 Gewichtsteile bezogen auf 100 Gewichtsteile von Wasser beträgt.

5. Viskose Zusammensetzung nach einem der Ansprüche 1 bis 4, wobei der Gehalt des polyvalenten Metallsalzes in der flüssigen Zusammensetzung 0,1 bis 4 Gewichtsteile bezogen auf 100 Gewichtsteile von Wasser beträgt.

6. Viskose Zusammensetzung nach einem der Ansprüche 1 bis 5, wobei diese ein kosmetisches Material, ein äußerliches Präparat für die Haut oder ein Toiletteartikel ist.

7. Viskose Zusammensetzung, umfassend (A) ein Alkyl-modifiziertes Carboxylgruppen-hältiges wasserlösliches Copolymer, das dadurch erhalten wurde, dass 100 Gewichtsteile einer (Meth)acrylsäure, 0,5 bis 5 Gewichtsteile eines (Meth)acrylsäurealkylesters, der eine Alkylgruppe mit 18 bis 24 Kohlenstoffatomen aufweist, und 0 bis 0,1 Gewichtsteile einer Verbindung, die zwei oder mehr ethylenisch ungesättigte Gruppen aufweist, einer Polymerisierung unterzogen wurden, (B) ein polyvalentes Metallsalz, (C) eine Base, (D) zumindest eine Art von Säure, die aus der aus Carbonsäure und Phosphoroxosäure bestehenden Gruppe ausgewählt ist, und (E) Wasser, wobei der Gehalt des Alkyl-modifizierten Carboxylgruppen-hältigen wasserlöslichen Copolymers in der flüssigen Zusammensetzung 0,1 bis 4 Gewichtsteile bezogen auf 100 Gewichtsteile von Wasser beträgt,
der pH 2,5 bis 6,5 ist und
der Grad an Viskosität bei 25 °C 1.000 bis 100.000 mPa•s beträgt.

8. Viskose Zusammensetzung nach Anspruch 7, wobei das polyvalente Metallsalz ein polyvalentes Metallsalz einer organischen Säure ist.

9. Viskose Zusammensetzung nach einem der Ansprüche 7 bis 8, wobei das Alkyl-modifizierte Carboxylgruppen-hältige wasserlösliche Copolymer, das polyvalente Metallsalz, die Base und die Carbonsäure und/oder die Phosphoroxosäure in Gehalten von 0,1 bis 4 Gew.-%, 0,1 bis 4 Gew.-%, 0,01 bis 6 Gew.-% bzw. 0,1 bis 4 Gew.-% enthalten sind.

10. Viskose Zusammensetzung nach einem der Ansprüche 7 bis 9, wobei diese ein kosmetisches Material, ein äußerliches Präparat für die Haut oder ein Toiletteartikel ist.

11. Verfahren zur Herstellung einer viskosen Zusammensetzung, umfassend den folgenden ersten bis dritten Schritt:
einen ersten Schritt zur Herstellung einer flüssigen Zusammensetzung, enthaltend ein Alkyl-modifiziertes Carboxylgruppen-hältiges wasserlösliches Copolymer, das erhalten wurde, indem 100 Gewichtsteile einer (Meth)acrylsäure, 0,5 bis 5 Gewichtsteile eines (Meth)acrylsäurealkylesters, der eine Alkylgruppe mit 18 bis 24 Kohlenstoffatomen aufweist, und 0 bis 0,1 Gewichtsteile einer Verbindung, die zwei oder mehr ethylenisch ungesättigte Gruppen aufweist, einer Polymerisierung unterzogen wurden, ein polyvalentes Metallsalz und Wasser,
wobei der Gehalt des Alkyl-modifizierten Carboxylgruppen-hältigen wasserlöslichen Copolymers in der flüssigen Zusammensetzung 0,1 bis 4 Gewichtsteile bezogen auf 100 Gewichtsteile von Wasser beträgt,
einen zweiten Schritt zur Herstellung einer alkalischen viskosen Zusammensetzung durch Zusetzen einer Base zur flüssigen Zusammensetzung, um den pH auf 7,0 oder mehr einzustellen, und
einen dritten Schritt zur Herstellung einer viskosen Zusammensetzung durch Zusetzen von zumindest einer Art von Säure, die aus der aus Carbonsäure und Phosphoroxosäure bestehenden Gruppe ausgewählt ist, zur alkalischen viskosen Zusammensetzung, um den pH auf 2,5 bis 6,5 einzustellen.

12. Verfahren zur Herstellung einer viskosen Zusammensetzung nach Anspruch 11, wobei das polyvalente Metallsalz ein polyvalentes Metallsalz einer organischen Säure ist.

13. Verfahren zur Herstellung einer viskosen Zusammensetzung nach Anspruch 11 oder 12, wobei das polyvalente Metallsalz ein polyvalentes Metallsalz eines Ascorbinsäurederivats ist.

14. Verfahren zur Herstellung einer viskosen Zusammensetzung nach einem der Ansprüche 11 bis 13, wobei der Gehalt des Alkyl-modifizierten Carboxylgruppen-hältigen wasserlöslichen Copolymers in der flüssigen Zusammensetzung 0,1 bis 3 Gewichtsteile bezogen auf 100 Gewichtsteile von Wasser beträgt.

15. Verfahren zur Herstellung einer viskosen Zusammensetzung nach einem der Ansprüche 11 bis 14, wobei der Gehalt des polyvalenten Metallsalzes in der flüssigen Zusammensetzung 0,1 bis 4 Gewichtsteile bezogen auf 100 Gewichtsteile von Wasser beträgt.

## Revendications

1. Composition visqueuse, obtenue en ajustant le pH d'une composition liquide contenant un copolymère hydrosoluble contenant un groupe carboxyle modifié par un alkyle, obtenu en laissant 100 parties en masse d'un acide (méth)acrylique, 0,5 à 5 parties en masse d'un ester alkylique d'acide (méth)acrylique ayant un groupe alkyle ayant 18 à 24 atomes de carbone, et 0 à 0,1 partie en masse d'un composé ayant deux groupes éthyléniquement insaturés ou plus subir une polymérisation, un sel de métal polyvalent et de l'eau jusqu'à 7,0 ou plus avec une base, puis en ajustant le pH entre 2,5 et 6,5 avec au moins un type d'acide choisi dans le groupe constitué par un acide carboxylique et un oxoacide phosphoreux, dans laquelle la teneur du copolymère hydrosoluble contenant un groupe carboxyle modifié par un alkyle dans la composition liquide est de 0,1 à 4 parties en masse pour 100 parties en masse d'eau.

2. Composition visqueuse selon la revendication 1, dans laquelle le sel de métal polyvalent est un sel de métal polyvalent d'un acide organique.

3. Composition visqueuse selon la revendication 1 ou 2, dans laquelle le sel de métal polyvalent est un sel de métal polyvalent d'un dérivé d'acide ascorbique.

4. Composition visqueuse selon l'une quelconque des revendications 1 à 3, dans laquelle la teneur en copolymère hydrosoluble contenant un groupe carboxyle modifié par un alkyle dans la composition liquide est de 0,1 à 3 parties en masse pour 100 parties en masse d'eau.

5. Composition visqueuse selon l'une quelconque des revendications 1 à 4, dans laquelle la teneur du sel de métal polyvalent dans la composition liquide est de 0,1 à 4 parties en masse pour 100 parties en masse d'eau.

6. Composition visqueuse selon l'une quelconque des revendications 1 à 5, qui est un matériau cosmétique, une préparation externe pour la peau ou un produit de toilette.

7. Composition visqueuse, comprenant (A) un copolymère hydrosoluble contenant un groupe carboxyle modifié par un alkyle, obtenu en laissant 100 parties en masse d'un acide (méth)acrylique, 0,5 à 5 parties en masse d'un ester alkylique d'acide (méth)acrylique ayant un groupe alkyle ayant 18 à 24 atomes de carbone, et 0 à 0,1 partie en masse d'un composé ayant deux groupes éthyléniquement insaturés ou plus subir une polymérisation, (B) un sel de métal polyvalent, (C) une base, (D) au moins un type d'acide choisi dans le groupe constitué par un acide carboxylique et un oxoacide phosphoreux, et (E) de l'eau, dans laquelle la teneur du copolymère hydrosoluble contenant un groupe carboxyle modifié par un alkyle dans la composition liquide est de 0,1 à 4 parties en masse pour 100 parties en masse d'eau.
le pH étant de 2,5 à 6,5, et
le degré de viscosité à 25°C étant compris entre 1 000 et 100 000 mPa·s.

8. Composition visqueuse selon la revendication 7, dans laquelle le sel de métal polyvalent est un sel de métal polyvalent d'un acide organique.

9. Composition visqueuse selon la revendication 7 ou 8, dans laquelle le copolymère hydrosoluble contenant un groupe carboxyle modifié par un alkyle, le sel de métal polyvalent, la base et l'acide carboxylique et/ou l'oxoacide phosphoreux sont contenus à des teneurs respectives de 0,1 à 4% en masse, 0,1 à 4% en masse, 0,01 à 6% en masse et 0,1 à 4% en masse.

10. Composition visqueuse selon l'une quelconque des revendications 7 à 9, qui est un matériau cosmétique, une préparation externe pour la peau ou un produit de toilette.

11. Procédé de production d'une composition visqueuse, comprenant les première à troisième étapes suivantes consistant à :
une première étape de préparation d'une composition liquide contenant un copolymère hydrosoluble contenant un groupe carboxyle modifié par un alkyle, obtenu en laissant 100 parties en masse d'un acide (méth)acrylique, 0,5 à 5 parties en masse d'un ester alkylique d'acide (méth)acrylique ayant un groupe alkyle ayant 18 à 24 atomes de carbone, et 0 à 0,1 partie en masse d'un composé ayant deux groupes éthyléniquement insaturés ou plus subir une polymérisation, un sel de métal polyvalent et de l'eau,
dans lequel la teneur du copolymère hydrosoluble contenant un groupe carboxyle modifié par un alkyle dans la composition liquide est de 0,1 à 4 parties en masse pour 100 parties en masse d'eau,
une deuxième étape pour préparer une composition visqueuse alcaline en ajoutant une base à la composition liquide pour ajuster le pH à 7,0 ou plus, et
une troisième étape pour préparer une composition visqueuse en ajoutant au moins un type d'acide choisi dans le groupe comprenant un acide carboxylique et un oxoacide phosphoreux à la composition visqueuse alcaline pour ajuster le pH entre 2,5 et 6,5.

12. Procédé de production d'une composition visqueuse selon la revendication 11, dans lequel le sel de métal polyvalent est un sel de métal polyvalent d'un acide organique.

13. Procédé de production d'une composition visqueuse selon la revendication 11 ou 12, dans lequel le sel de métal polyvalent est un sel de métal polyvalent d'un dérivé d'acide ascorbique.

14. Procédé de production d'une composition visqueuse selon l'une quelconque des revendications 11 à 13, dans lequel la teneur du copolymère hydrosoluble contenant un groupe carboxyle modifié par un alkyle dans la composition liquide est de 0,1 à 3 parties en masse pour 100 parties en masse d'eau.

15. Procédé de production d'une composition visqueuse selon l'une quelconque des revendications 11 à 14, dans lequel la teneur du sel de métal polyvalent dans la composition liquide est de 0,1 à 4 parties en masse pour 100 parties en masse d'eau.
